# EUROPEAN PATENT APPLICATION

(11) **EP 4 653 540 A1**
(43) Date of publication of application: **26.11.2025**
(21) Application number: 24744733.7
(22) Date of filing: 19.01.2024
(51) Int. Cl.: C12P 19/00, C12N 1/15, C12N 1/19, C12N 1/21, C12N 9/24, C12N 15/56, C13K 13/00

(54) **FRUCTOSYLATED MALTITOL, AND METHOD FOR PRODUCING SAME**

(30) Priority: 20.01.2023 JP 2023007058
(71) Applicant: Kyoto Prefectural Public University Corporation, Kyoto-shi, Kyoto 602-8566 (JP); MEIJI CO., LTD, Chuo-ku Tokyo 104-8306 (JP)
(72) Inventor: TANAKA Shun-ichi, Kyoto-shi, Kyoto 606-8522 (JP); YANO Mamiko, Kyoto-shi, Kyoto 606-8522 (JP); UENO Keiichi, Tokyo 136-0075 (JP); MIYATAKE Takumi, Hachioji-shi, Tokyo 192-0919 (JP)
(74) Representative: TBK
(86) International application number: PCT/JP2024/001393
(87) International publication number: WO 2024/154813

(57) **Abstract**

An object of the present invention is to provide a novel fructosylated maltitol and a method for producing the same. According to the present invention, there is provided a method for producing a fructosylated maltitol, comprising the step of contacting β-fructofuranosidase with sucrose and maltitol (contact step), wherein the β-fructofuranosidase has a sequence identity of 60% or more with the amino acid sequence set forth in SEQ ID NO: 1 and has β-fructofuranosidase activity. The present invention is advantageous in that it is possible to simply and efficiently produce a fructosylated maltitol.

## Description

### CROSS-REFERENCE TO RELATED APPLICATION

The present application enjoys the benefit of priority from the prior Japanese Patent Application No. 2023-7058 filed on January 20, 2023, the entire disclosure of which is incorporated herein by reference.

### TECHNICAL FIELD

The present invention relates to a fructosylated maltitol and a method for producing the same.

### BACKGROUND ART

Since an gut microbiota has a significant influence on human health, improving the microbial composition which forms the gut microbiota provides a health promoting action. Prebiotics refer to indigestible food ingredients that selectively promote the proliferation and activity of specific beneficial bacteria, such as *Bifidobacterium,* which live in the intestinal environment. The prebiotics are known as a more practical and efficient method for manipulating the gut microbiota because target bacteria already live symbiotically in the intestines, unlike probiotics that provide a health promoting action upon intake of exogenous bacteria (Non-Patent Document 1).

Sugar alcohols are low-calorie sweeteners that reach the large intestine without being digested or absorbed, and are expected to be function as prebiotics.

On the other hand, there is a demand for development of prebiotic materials that are superior in terms of functions and physical properties. As a method for fructosylating a sugar alcohol, for example, a method using β-fructofuranosidase derived from a microorganism belonging to the genus Schwanniomyces is known (Non-Patent Document 2).

### Reference List

### Non-Patent Documents

Non-Patent Document 1: Gibson GR, et al., J Nutr., 1995; 125(6): 1401-1412.
Non-Patent Document 2: Piedrabuena D et al., Appl Microbiol Biotechnol. 2016; 100(20): 8769-8778.

### SUMMARY OF THE INVENTION

### Technical Problem

An object of the present invention is to provide a novel fructosylated maltitol and a method for producing the same.

### Solution to Problem

The present inventors have now found that a fructosylated maltitol is produced by contacting β-fructofuranosidase derived from *Aspergillus fijiensis* with sucrose and maltitol. The present inventors have also found that the β-fructofuranosidase has substrate specificity for maltitol among sugar alcohols. Further, the present inventors have found that the production amount of the fructosylated maltitol is increased by increasing a ratio of the maltitol to the sucrose at the time of the contact. The present invention is based on the findings.

According to the present invention, the following inventions are provided.
[1] A method for producing a fructosylated maltitol, comprising the step of contacting (A) β-fructofuranosidase or (B) a polypeptide comprising a part thereof, or
   (C) a culture of a transformant comprising either or both of the β-fructofuranosidase and the polypeptide, with sucrose and maltitol (contact step),
      wherein the (A) β-fructofuranosidase has a sequence identity of 60% or more with the amino acid sequence set forth in SEQ ID NO: 1 and has β-fructofuranosidase activity,
      wherein the (B) polypeptide comprises an enzymatically active moiety of the amino acid sequence of β-fructofuranosidase having the amino acid sequence set forth in SEQ ID NO: 1 and has β-fructofuranosidase activity, and
      wherein the (C) culture of the transformant is a culture of a transformant obtained by introducing into a host an expression vector comprising a polynucleotide encoding the (A) β-fructofuranosidase or a polynucleotide encoding the (B) polypeptide.
[2] The method according to [1], wherein the β-fructofuranosidase has substrate specificity for maltitol among sugar alcohols.
[3] The method according to [1] or [2], wherein a ratio of the amount or concentration of the maltitol to the sucrose at the start of contact is 1 or more.
[4] The method according to any one of [1] to [3], wherein a temperature of the contact step is 40°C to 60°C.
[5] The method according to any one of [1] to [4], wherein a period of time of the contact step is 8 hours or more.
[6] The method according to any one of [1] to [5], wherein the host is selected from the group consisting of bacteria, yeasts, molds and filamentous fungi.
[7] The method according to any one of [1] to [6], wherein the β-fructofuranosidase is derived from a microorganism of the genus *Aspergillus.*
[8] The method according to any one of [1] to [7], wherein the β-fructofuranosidase is derived from *Aspergillus fijiensis.*
[9] An enzyme preparation for use in production of a fructosylated maltitol, comprising β-fructofuranosidase or a polypeptide comprising a part thereof, or a culture of a transformant comprising either or both of the β-fructofuranosidase and the polypeptide,
   wherein the β-fructofuranosidase has a sequence identity of 60% or more with the amino acid sequence set forth in SEQ ID NO: 1 and has β-fructofuranosidase activity,
   wherein the polypeptide comprises an enzymatically active moiety of the amino acid sequence of β-fructofuranosidase having the amino acid sequence set forth in SEQ ID NO: 1 and has β-fructofuranosidase activity, and
   wherein the culture of the transformant is a culture of a transformant obtained by introducing into a host an expression vector comprising a polynucleotide encoding the β-fructofuranosidase or a polynucleotide encoding the polypeptide.
[10] A fructosylated maltitol which is maltitol to which one or two molecules of fructose is/are bound.
[11] The fructosylated maltitol according to [10], wherein the maltitol to which one molecule of fructose is bound is represented by Formula (I) below.
[12] The fructosylated maltitol according to [10], wherein the maltitol to which two molecules of fructose are bound is represented by Formula (II) below.
[13] An enzyme reaction composition comprising: as an enzyme, (D) β-fructofuranosidase or (E) a polypeptide comprising a part thereof, or (F) a culture of a transformant comprising either or both of the β-fructofuranosidase and the polypeptide; and, as a substrate, sucrose and maltitol,
   wherein a proportion of the fructosylated maltitol to all sugars in the composition is 5% or more,
   wherein the (D) β-fructofuranosidase has a sequence identity of 60% or more with the amino acid sequence set forth in SEQ ID NO: 1 and has β-fructofuranosidase activity,
   wherein the (E) polypeptide comprises an enzymatically active moiety of the amino acid sequence of β-fructofuranosidase having the amino acid sequence set forth in SEQ ID NO: 1 and has β-fructofuranosidase activity, and
   wherein the (F) culture of the transformant is a culture of a transformant obtained by introducing into a host an expression vector comprising a polynucleotide encoding the (D) β-fructofuranosidase or a polynucleotide encoding the (E) polypeptide.
[14] Use of β-fructofuranosidase for producing a fructosylated maltitol, wherein the β-fructofuranosidase has a sequence identity of 60% or more with the amino acid sequence set forth in SEQ ID NO: 1 and has β-fructofuranosidase activity.

The present invention is advantageous in that it is possible to simply and efficiently produce a fructosylated maltitol. The present invention is also advantageous in that, since the β-fructofuranosidase has substrate specificity for maltitol among sugar alcohols, byproducts are less likely to be generated when the fructosylated maltitol is produced.

### BRIEF DESCRIPTION OF THE DRAWINGS

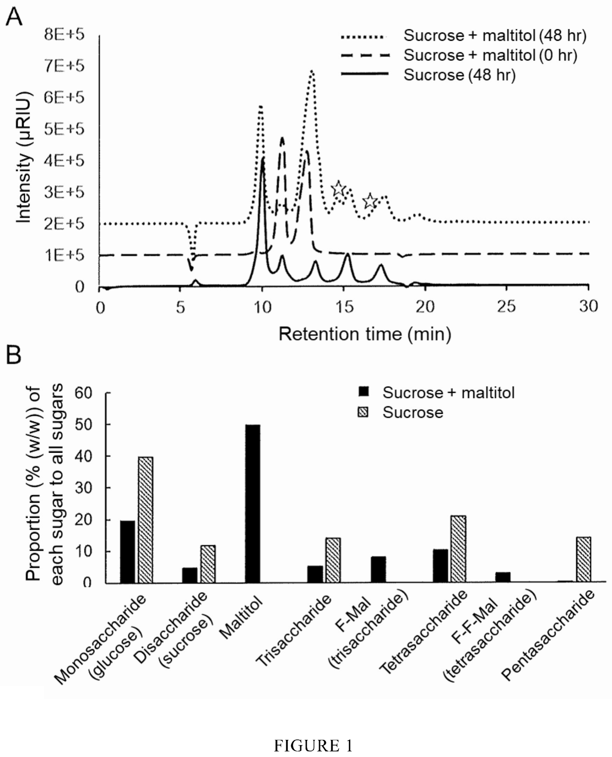

[FIG. 1] FIG. 1A shows results (chromatogram) of HPLC analysis of a reaction solution when maltitol was used as a sugar alcohol. The solid line in this figure indicates the case where the substrate was sucrose only (48 hours), the dashed line indicates the case where the substrate was sucrose and maltitol (sucrose + maltitol) (0 hours), and the dotted line indicates the case where the substrate was sucrose and maltitol (sucrose + maltitol) (48 hours) (the time in parentheses indicates a reaction time). The stars in this figure represent maltitol to which one molecule of fructose was bound (left star) and maltitol to which two molecules of fructose were bound (right star), respectively. FIG. 1B shows a proportion of each sugar to all sugars in the reaction solution when maltitol was used as the sugar alcohol.

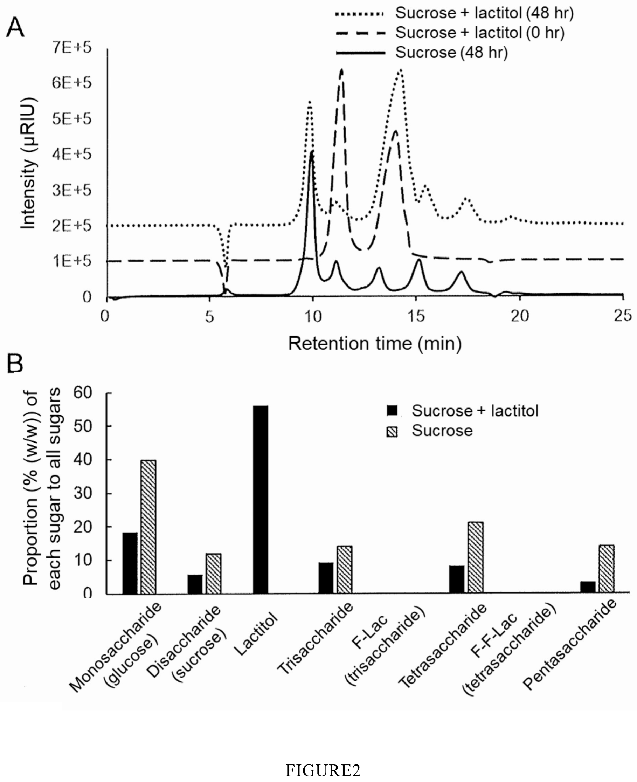

[FIG. 2] FIG. 2A shows results (chromatogram) of HPLC analysis of a reaction solution when lactitol was used as the sugar alcohol. The solid line in this figure indicates the case where the substrate was sucrose only (48 hours), the dashed line indicates the case where the substrate was sucrose and lactitol (sucrose + lactitol) (0 hours), and the dotted line indicates the case where the substrate was sucrose and lactitol (sucrose + lactitol) (48 hours) (the time in parentheses indicates a reaction time). FIG. 2B shows a proportion of each sugar to all sugars in the reaction solution when lactitol was used as the sugar alcohol.

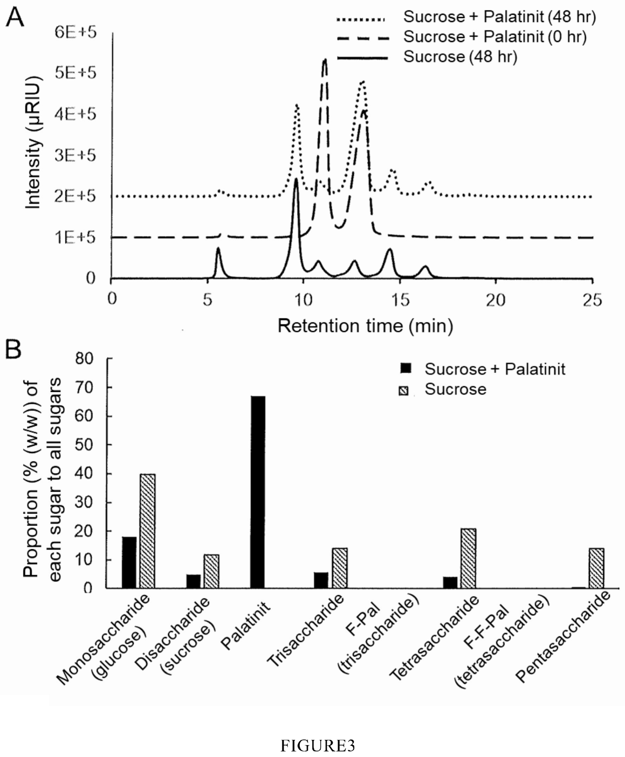

[FIG. 3] FIG. 3A shows results (chromatogram) of HPLC analysis of a reaction solution when Palatinit was used as the sugar alcohol. The solid line in this figure indicates the case where the substrate was sucrose only (48 hours), the dashed line indicates the case where the substrate was sucrose and Palatinit (sucrose + Palatinit) (0 hours), and the dotted line indicates the case where the substrate was sucrose and Palatinit (sucrose + Palatinit) (48 hours) (the time in parentheses indicates a reaction time). FIG. 3B shows a proportion of each sugar to all sugars in the reaction solution when Palatinit was used as the sugar alcohol.

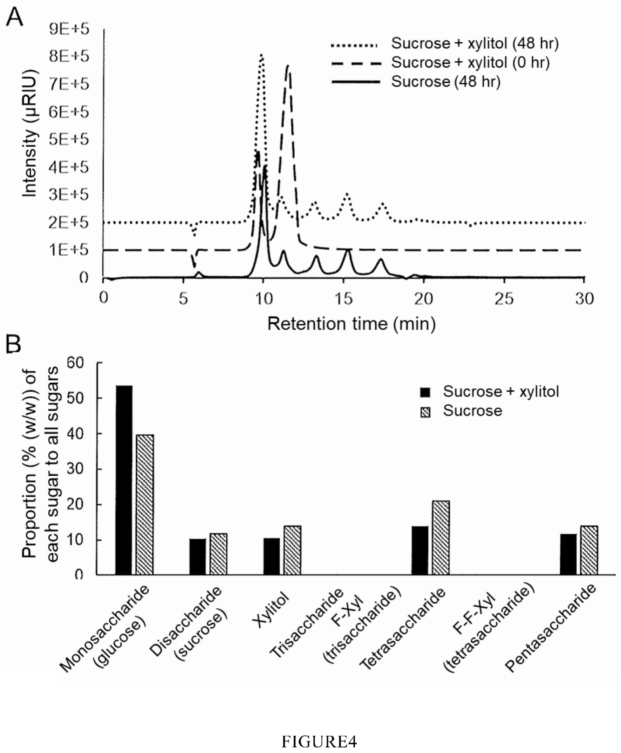

[FIG. 4] FIG. 4A shows results (chromatogram) of HPLC analysis of a reaction solution when xylitol was used as the sugar alcohol. The solid line in this figure indicates the case where the substrate was sucrose only (48 hours), the dashed line indicates the case where the substrate was sucrose and xylitol (sucrose + xylitol) (0 hours), and the dotted line indicates the case where the substrate was sucrose and xylitol (sucrose + xylitol) (48 hours) (the time in parentheses indicates a reaction time). FIG. 4B shows a proportion of each sugar to all sugars in the reaction solution when xylitol was used as the sugar alcohol.
[FIG. 5] FIG. 5A shows proportions of each sugar to all sugars in reaction solutions when ratios of maltitol to sucrose used were 1:1, 1:2 and 1:3. FIG. 5B shows proportions of each FOS to all FOSs in reaction solutions when ratios of maltitol to sucrose used were 1:1, 1:2 and 1:3.
[FIG. 6] FIG. 6 shows changes in proportions of maltitol to which one molecule of fructose was bound (×), maltitol to which two molecules of fructose were bound (•), and the total thereof (◆) to all sugars at each reaction time when maltitol was used as the sugar alcohol.
[FIG. 7] FIG. 7 shows a ¹H NMR spectrum (600 MHz) of 1-OBz in CDCl₃.
[FIG. 8] FIG. 8 shows an extended ¹H NMR spectrum (600 MHz) of 1-OBz in CDCl₃ (6.1 to 3.3 ppm).
[FIG. 9] FIG. 9 shows an extended ¹H NMR spectrum (600 MHz) of 1-OBz in CDCl₃ (8.5 to 6.7 ppm).
[FIG. 10] FIG. 10 shows a ¹³C NMR spectrum (upper) and a DEPT 135 spectrum (lower) (150 MHz) of 1-OBz in CDCl₃.
[FIG. 11] FIG. 11 shows a COSY NMR spectrum of 1-OBz in CDCl₃.
[FIG. 12] FIG. 12 shows a NOESY NMR spectrum of 1-OBz in CDCl₃.
[FIG. 13] FIG. 13 shows an HSQC NMR spectrum of 1-OBz in CDCl₃.
[FIG. 14] FIG. 14 shows an HMBC NMR spectrum of 1-OBz in CDCl₃.

### DETAILED DESCRIPTION OF THE INVENTION

### <<Definition>>

In the present specification, the "fructooligosaccharides (FOSs; sometimes referred to herein as "FOSs")" refer to oligosaccharides in which one to three molecules of fructose is/are bound to sucrose. The FOSs include 1-kestose in which one molecule of fructose is bound by a β-2,1 bond to the fructose residue of sucrose (sometimes referred to herein as "GF2" or "trisaccharide FOS"), nystose in which two molecules of fructose are bound by a β-2,1 bond to the fructose residue of sucrose (sometimes referred to herein as "GF3" or "tetrasaccharide FOS"), and 1-fructofuranosyl-D-nystose in which three molecules of fructose are bound by a β-2,1 bond to the fructose residue of sucrose (sometimes referred to herein as "GF4" or "pentasaccharide FOS"). The FOS is synthesized by β-fructofuranosidase.

In the present invention, the "fructosylated maltitol" is defined as a molecule in which fructose is bound to maltitol as a sugar alcohol. The number of fructose molecules bound to maltitol is at least 1 or 2, and the upper limit thereof can be 3, but may be 3 or more.

The structure of a fructosylated maltitol in which one molecule of fructose is bound to maltitol as determined in the Examples below (sometimes described herein as "F-Mal") is shown in Formula (I). As shown in Formula (I), the 2-position of fructose is bound to the 6-position of the glucose moiety of maltitol.

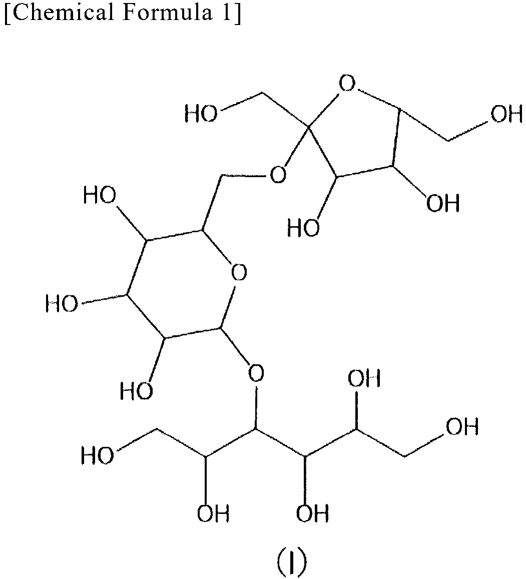

The structure presumed as a fructosylated maltitol in which two molecules of fructose are bound to maltitol (sometimes described herein as "F-F-Mal") is shown in Formula (II). It is considered that the 2-position of one molecule of fructose is bound to the 6-position of the glucose moiety of maltitol, and that the 2-position of another molecule of fructose is bound to the 1-position of this fructose, as shown in Formula (II).

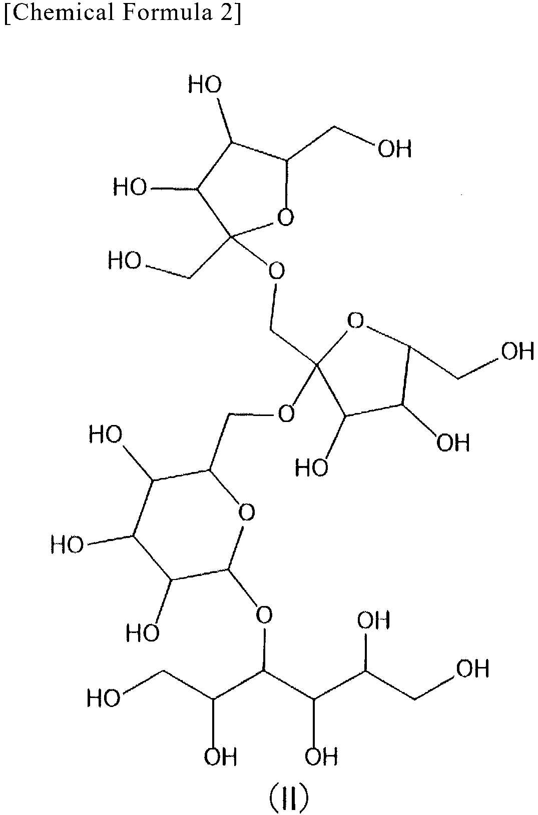

In the case of a fructosylated maltitol in which three or more molecules of fructose are bound to maltitol, it is considered that the 2-position of still another molecule of fructose is bound to the 1-position of fructose at a terminal of the fructosylated maltitol. Taking, as an example, a fructosylated maltitol in which three molecules of fructose are bound to maltitol, it is presumed that the 2-position of another molecule of fructose is bound to the 1-position of fructose at a terminal of F-F-Mal (fructose further bound to the first fructose bound to maltitol), as shown in Formula (III).

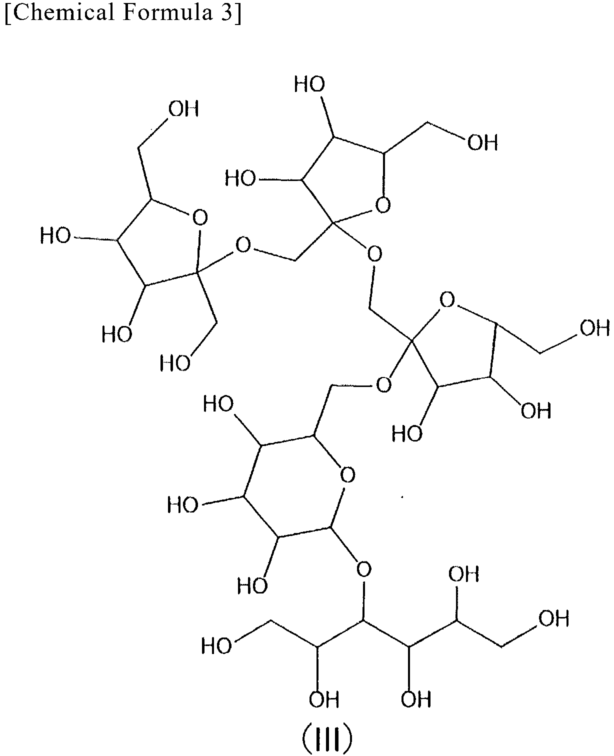

### <<Method for producing fructosylated maltitol>>

According to the present invention, a method for producing a fructosylated maltitol is provided.

According to the production method of the present invention, there is provided a method for producing a fructosylated maltitol, comprising the step of contacting (A) β-fructofuranosidase with sucrose and maltitol (contact step), wherein the (A) β-fructofuranosidase has a sequence identity of 60% or more with the amino acid sequence set forth in SEQ ID NO: 1 and has β-fructofuranosidase activity.

In the present invention, the "β-fructofuranosidase" refers to an enzyme belonging to the GH32 family, which has sucrose hydrolysis activity among GH families (glycoside hydrolase families; referring to a group of homologous proteins with high sequence similarity among enzymes that hydrolyze glycosidic bonds of sugars). The term "β-fructofuranosidase" is sometimes used interchangeably with the term "β-fructofuranosidase," "fructosyltransferase," "fructosyltransferase," "saccharase," "β-D-fructofuranosidase," "β-D-fructofuranosidase," "invertase," "invertase," or "invertin," and all of these terms have the same meaning.

The β-fructofuranosidase used in the production method of the present invention can consist of an amino acid sequence having a sequence identity of 60% or more (preferably, 65% or more, 70% or more, 75% or more, 80% or more, 85% or more, 90% or more, 93% or more, 95% or more, 98% or more, or 99% or more) with the amino acid sequence of β-fructofuranosidase set forth in SEQ ID NO: 1. The term "identity," as used herein, refers to the degree of identity when the sequences to be compared are appropriately aligned, and means the appearance rate (%) of exact amino acid matches between the sequences. For example, the presence of gaps and the properties of amino acids in the sequences are taken into consideration in the calculation of the identity (Wilbur, Natl. Acad. Sci. U.S.A. 80:726-730 (1983)). The alignment can be performed, for example, by using any algorithm. Specifically, publicly available homology search software such as BLAST (Basic local alignment search tool) (Altschul et al., J. Mol. Biol. 215:403-410 (1990)), FASTA (Peasron et al., Methods in Enzymology 183:63-69 (1990)), and Smith-Waterman (Meth. Enzym., 164, 765 (1988)) can be used. Further, the identity can be calculated, for example, using a publicly available homology search program as described above. For example, the identity can be calculated using default parameters in the homology algorithm BLAST (https://blast.ncbi.nlm.nih.gov/Blast.cgi) of the National Center for Biotechnology Information (NCBI).

The β-fructofuranosidase used in the production method of the present invention also consists of an amino acid sequence which has a sequence identity of 60% or more with the amino acid sequence of β-fructofuranosidase set forth in SEQ ID NO: 1 and has β-fructofuranosidase activity. In the present invention, the phrase "having β-fructofuranosidase activity" can be defined as the amino acid sequence having about 70% or more of the activity, about 80% or more of the activity, about 90% or more of the activity, about 95% or more of the activity, or about 100% or more of the activity of β-fructofuranosidase consisting of the amino acid sequence set forth in SEQ ID NO: 1 under conditions of a temperature of 50 to 55°C and a pH of 5.5. The β-fructofuranosidase activity can be evaluated from a value obtained, for example, by mixing a solution containing the protein or polypeptide to be measured with a solution containing sucrose, incubating the mixed solution, and measuring the amount of glucose generated by an enzyme reaction using high performance liquid chromatography (HPLC), thin layer chromatography (TLC), various glucose quantification kits and the like, and from the amount of enzyme used in the reaction.

Also, the β-fructofuranosidase used in the production method of the present invention is typically a wild-type enzyme, can be derived from any organism such as a microorganism, a fungus, or a plant, and is preferably derived from a microorganism belonging to the genus *Aspergillus,* and more preferably derived from *Aspergillus fijiensis.* However, the present invention does not preclude use of β-fructofuranosidase into which an artificial mutation is introduced in the production method of the present invention.

Examples of the β-fructofuranosidase derived from a microorganism belonging to the genus *Aspergillus* include those indicated in Table 1.

**[Table 1]**

| Table 1: Examples of β-fructofuranosidase derived from microorganism (bacterial species) belonging to the genus *Aspergillus.* which has sequence identity of 60% or more with amino acid sequence of β-fructofuranosidase set forth in SEQ ID NO: 1 | | |
|---|---|---|
| Bacterial species (origin) | Accession No. | Sequence identity (%) |
| *Aspergillus aculeatus* | ANF99482.1 | 99.69 |
| *Aspergillus japonicus* | 3LF7_A | 99.53 |
| *Aspergillus japonicus* | ADK46938.1 | 99.39 |
| *Aspergillus japonicus* | 3LDK_A | 99.37 |
| *Aspergillus fijiensis* CBS 313.89 | XP_040799211.1 | 99.08 |
| *Aspergillus brunneoviolaceus* CBS 621.78 | XP_025443891.1 | 98.47 |
| *Aspergillus aculeatinus* CBS 121060 | XP_025508898.1 | 97.40 |
| *Aspergillus japonicus* | ABD97344.1 | 97.40 |
| *Aspergillus aculeatus* ATCC 16872 | XP_020051300.1 | 96.18 |
| *Aspergillus uvarum* CBS 121591 | XP_025487796.1 | 93.10 |
| *Aspergillus indologenus* CBS 114.80 | PYI32185.1 | 92.98 |
| *Aspergillus japonicus* CBS 114.51 | XP_025532678.1 | 91.85 |
| *Aspergillus violaceofuscus* CBS 115571 | PVI19045.1 | 90.37 |
| *Aspergillus homomorphus* CBS 101889 | XP_025551517.1 | 83.78 |
| *Aspergillus saccharolyticus* JOP 1030-1 | XP_025428139.1 | 81.69 |
| *Aspergillus costaricaensis* CBS 115574 | XP_025540508.1 | 64.98 |
| *Aspergillus luchuensis* IFO 4308 | 5XH8_A | 64.61 |
| *Aspergillus ellipticus* CBS 707.79 | PYH88239.1 | 64.49 |
| *Aspergillus niger* | AHC54391.1 | 63.94 |
| *Aspergillus costaricaensis* CBS 115574 | XP_025540508.1 | 63.94 |

The β-fructofuranosidase used in the production method of the present invention may also be β-fructofuranosidase having substrate specificity for maltitol among sugar alcohols.

The β-fructofuranosidase used in the production method of the present invention may also be β-fructofuranosidase having one or more modifications in the amino acid sequence of β-fructofuranosidase set forth in SEQ ID NO: 1 and having β-fructofuranosidase activity. The modification may be one or more modifications selected from the group consisting of deletions, substitutions, insertions and additions. The modifications may also be a plurality of modifications of the same type (e.g., a plurality of substitutions) or a plurality of modifications of different types (e.g., a combination of one or more deletions and one or more substitutions). The number of amino acids to be modified can be, for example, 1 to 300, 1 to 275, 1 to 250, 1 to 225, 1 to 200, 1 to 175, 1 to 150, 1 to 125, 1 to 100, 1 to 90, 1 to 80, 1 to 70, 1 to 60, 1 to 50, 1 to 40, 1 to 30, 1 to 20, or 1 to 10, and is preferably 1 to 60, 1 to 50, 1 to 40, 1 to 30, 1 to 20, or 1 to 10, and more preferably 1 to several, 1 to 9, 1 to 8, 1 to 7, 1 to 6, 1 to 5, 1 to 4, 1 to 3, 1 to 2, or 1. The number of amino acids to be modified can be equivalent to the number of mutations occurring by a known method such as site-specific mutagenesis, or equivalent to the number of naturally occurring mutations.

The modification of an amino acid in the amino acid sequence of β-fructofuranosidase set forth in SEQ ID NO: 1 may be a conservative modification (e.g., conservative mutation). The term "conservative modification" or "conservative mutation" means that one or more amino acids are modified or mutated in such a manner that the functions of the protein are not substantially modified. The substitution of the amino acid may also be a conservative substitution. The "conservative substitution" means that one or more amino acids are substituted with other amino acids and/or amino acid derivatives in such a manner that the functions of the protein are not substantially modified. In the conservative substitution, the amino acid to be substituted and the substituted amino acid are preferably similar in properties and/or functions, for example. Specifically, these amino acids are preferably similar in chemical properties, such as hydrophobicity and hydrophilicity indices, polarity, and charge, or physical properties, such as secondary structure. Thus, amino acids or amino acid derivatives having similar properties and/or functions are known in the art. Examples of non-polar amino acids (hydrophobic amino acids) include alanine, valine, isoleucine, leucine, proline, tryptophan, phenylalanine, and methionine. Examples of polar amino acids (neutral amino acids) include glycine, serine, threonine, tyrosine, glutamine, asparagine, and cysteine. Examples of positively charged amino acids (basic amino acids) include arginine, histidine, and lysine, and examples of negatively charged amino acids (acidic amino acids) include aspartic acid and glutamic acid.

According to the production method of the present invention, there is provided a method for producing a fructosylated maltitol, comprising the step of contacting (B) a polypeptide comprising a part of the (A) β-fructofuranosidase with sucrose and maltitol (contact step), wherein the (B) polypeptide comprises an enzymatically active moiety of the amino acid sequence of β-fructofuranosidase having the amino acid sequence set forth in SEQ ID NO: 1 and has β-fructofuranosidase activity.

The polypeptide used in the production method of the present invention typically comprises at least a site (region) having β-fructofuranosidase activity. Examples of the site having β-fructofuranosidase activity in the β-fructofuranosidase used in the production method of the present invention include a site corresponding to the 20^{th} to 654^{th} positions from an amino terminus of the amino acid sequence set forth in SEQ ID NO: 1 (region excluding an N-terminal signal sequence); a site corresponding to the 50^{th} to 558^{th} positions from an amino terminus of the amino acid sequence set forth in SEQ ID NO: 1 (region characteristic of an enzyme belonging to glycosyl hydrolase family 32); a site corresponding to the 59^{th} to 428^{th} positions from an amino terminus of the amino acid sequence set forth in SEQ ID NO: 1 (region characteristic of an N-terminal sequence of an enzyme belonging to glycosyl hydrolase family 32); a site corresponding to the 60^{th} position from an amino terminus of the amino acid sequence set forth in SEQ ID NO: 1 (catalytic center amino acid Asp60); a site corresponding to the 191^{st} position from an amino terminus of the amino acid sequence set forth in SEQ ID NO: 1 (catalytic center amino acid Asp191); and a site corresponding to the 292^{nd} position from an amino terminus of the amino acid sequence set forth in SEQ ID NO: 1 (catalytic center amino acid Glu292). The upper limit value (the limit value or less, or less than the limit value) of the total length of the polypeptide including the site (region) having β-fructofuranosidase activity can be, for example, 800-amino acid length, 775-amino acid length, 750-amino acid length, 725-amino acid length, 700-amino acid length, 690-amino acid length, 685-amino acid length, 680-amino acid length, 675-amino acid length, 670-amino acid length, 665-amino acid length, 660-amino acid length, 659-amino acid length, 658-amino acid length, 657-amino acid length, 656-amino acid length, 655-amino acid length, 654-amino acid length, 653-amino acid length, 652-amino acid length, 651-amino acid length, 650-amino acid length, 649-amino acid length, 648-amino acid length, 647-amino acid length, 646-amino acid length, 645-amino acid length, 644-amino acid length, 643-amino acid length, 642-amino acid length, 641-amino acid length, 640-amino acid length, 639-amino acid length, 638-amino acid length, 637-amino acid length, 636-amino acid length, or 635-amino acid length. Also, the total length of the polypeptide used in the production method of the present invention can be adjusted arbitrarily, as long as it comprises the site having β-fructofuranosidase activity.

According to the production method of the present invention, there is provided a method for producing a fructosylated maltitol, comprising the step of contacting (C) a culture of a transformant comprising either or both of the (A) β-fructofuranosidase and the (B) polypeptide, with sucrose and maltitol (contact step), wherein the (C) culture of the transformant is a culture of a transformant obtained by introducing into a host an expression vector comprising a polynucleotide encoding the (A) β-fructofuranosidase or a polynucleotide encoding the (B) polypeptide.

The culture of the transformant used in the production method of the present invention is obtained by culturing a transformant obtained by introducing into an appropriate host a polynucleotide encoding the (A) β-fructofuranosidase or the (B) polypeptide. The vector to be introduced into the host may be a vector into which the polypeptide is incorporated. Examples of the vector can include phage vectors, plasmid vectors, cosmids, and phagemids, and the vector can be appropriately selected depending on the host and manipulability. Examples of the host can include bacteria such as *E. coli* and *Bacillus subtilis,* yeasts, molds, and filamentous fungi, and the host can be appropriately selected depending on the type and manipulability of the recombinant vector.

In the production method of the present invention, a ratio of the amount or concentration of the maltitol to the sucrose at the start of contact is not particularly limited, and can be, for example, 1 or more. From the viewpoint of increasing the production amount of the fructosylated maltitol, the ratio is preferably 2 or more, and more preferably 3 or more.

In the production method of the present invention, a temperature of the contact step is not particularly limited as long as it is a temperature at which the (A) β-fructofuranosidase, the (B) polypeptide, or the (C) culture of the transformant has enzymatic activity, and can be, for example, 40°C to 60°C, preferably 45°C to 55°C.

In the production method of the present invention, a period of time of the contact step can be appropriately set depending on the target yield of the fructosylated maltitol to be produced, and can be, for example, 2 hours or more, 3 hours or more, 4 hours or more, 5 hours or more, 6 hours or more, 7 hours or more, 8 hours or more, 9 hours or more, 10 hours or more, 11 hours or more, 12 hours or more, 13 hours or more, 14 hours or more, 15 hours or more, 16 hours or more, 17 hours or more, 18 hours or more, 19 hours or more, 21 hours or more, 22 hours or more, 23 hours or more, 24 hours or more, 25 hours or more, 26 hours or more, 27 hours or more, 28 hours or more, 29 hours or more, 30 hours or more, 31 hours or more, 32 hours or more, 33 hours or more, 34 hours or more, 35 hours or more, 36 hours or more, 37 hours or more, 38 hours or more, 39 hours or more, 40 hours or more, 41 hours or more, 42 hours or more, 43 hours or more, 44 hours or more, 45 hours or more, 46 hours or more, 47 hours or more, 48 hours or more, 49 hours or more, 50 hours or more, 51 hours or more, 52 hours or more, 53 hours or more, 54 hours or more, 55 hours or more, 60 hours or more, 65 hours or more, 70 hours or more, 75 hours or more, 80 hours or more, 85 hours or more, 90 hours or more, 95 hours or more, or 100 hours or more. The period of time of the contact step can also be set to any period of time in a range of 2 hours or more and 100 hours or less, 10 hours or more and 100 hours or less, 20 hours or more and 100 hours or less, 30 hours or more and 100 hours or less, 40 hours or more and 100 hours or less, or 50 hours or more and 100 hours or less, and, from the viewpoint of increasing the production efficiency of the fructosylated maltitol, is preferably 55 hours or more and 100 hours or less.

The production method of the present invention can comprise the step of concentrating a sugar liquid containing the fructosylated maltitol (concentration step) after the contact step. The concentration step can be carried out according to a usual method.

The production method of the present invention can comprise the step of purifying the fructosylated maltitol in the sugar liquid or a concentrate liquid (purification step) after the contact step or the concentration step. The purification step can be carried out according to a usual method.

### <<Enzyme preparation>>

According to the present invention, there is also provided an enzyme preparation for use in production of a fructosylated maltitol, comprising the (A) β-fructofuranosidase, the (B) polypeptide, or the (C) culture of the transformant. The enzyme preparation of the present invention can be implemented according to the descriptions about the production method of the present invention.

### <<Fructosylated maltitol>>

According to the present invention, there is also provided a fructosylated maltitol, characterized by being maltitol to which one, two or three molecules of fructose is/are bound.

The fructosylated maltitol of the present invention is a fructosylated maltitol represented by Formula (I) above when one molecule of fructose is bound to maltitol, in which the 2-position of fructose is bound to the 6-position of the glucose moiety of maltitol.

The fructosylated maltitol of the present invention is a fructosylated maltitol represented by Formula (II) above when two molecules of fructose are bound to maltitol, and it is considered that the 2-position of one molecule of fructose is bound to the 6-position of the glucose moiety of maltitol, and that the 2-position of another molecule of fructose is bound to the 1-position of this fructose.

The fructosylated maltitol of the present invention is a fructosylated maltitol represented by Formula (III) above when three molecules of fructose are bound to maltitol, and it is considered that the 2-position of another molecule of fructose is bound to the 1-position of fructose at a terminal of F-F-Mal.

The fructosylated maltitol of the present invention can be implemented according to the descriptions about the production method of the present invention.

### <<Enzyme reaction composition containing fructosylated maltitol>>

According to the present invention, there is also provided an enzyme reaction composition comprising: β-fructofuranosidase as an enzyme; and sucrose and maltitol as a substrate.

According to the enzyme reaction composition according to the present invention, there is provided an enzyme reaction composition comprising: (D) β-fructofuranosidase as an enzyme; and sucrose and maltitol as a substrate, wherein a proportion of the fructosylated maltitol to all sugars in the composition is 5% or more, and wherein the (D) β-fructofuranosidase has a sequence identity of 60% or more with the amino acid sequence set forth in SEQ ID NO: 1 and has β-fructofuranosidase activity.

According to the enzyme reaction composition according to the present invention, there is also provided an enzyme reaction composition comprising: (E) a polypeptide comprising a part of the (D) β-fructofuranosidase as an enzyme; and sucrose and maltitol as a substrate, wherein a proportion of the fructosylated maltitol to all sugars in the composition is 5% or more, and wherein the (E) polypeptide comprises an enzymatically active moiety of the amino acid sequence of β-fructofuranosidase having the amino acid sequence set forth in SEQ ID NO: 1 and has β-fructofuranosidase activity.

According to the enzyme reaction composition according to the present invention, there is further provided an enzyme reaction composition comprising: (F) a culture of a transformant comprising either or both of the (D) β-fructofuranosidase and the (E) polypeptide as an enzyme; and sucrose and maltitol as a substrate, wherein a proportion of the fructosylated maltitol to all sugars in the composition is 5% or more, and wherein the (F) culture of the transformant is a culture of a transformant obtained by introducing into a host an expression vector comprising a polynucleotide encoding the (D) β-fructofuranosidase or a polynucleotide encoding the (E) polypeptide.

The enzyme reaction composition of the present invention has a proportion of the fructosylated maltitol to all sugars in the composition of 5% by mass or more, but further can be 6% by mass or more, 7% by mass or more, 8% by mass or more, 9% by mass or more, or 10% by mass or more.

The enzyme reaction composition of the present invention can be produced according to the descriptions about the production method of the present invention, in addition to the above description.

### <<Method for fructosylating maltitol>>

According to the present invention, a method for fructosylating maltitol is provided.

According to the fructosylation method of the present invention, there is provided a method for fructosylating maltitol, comprising the step of contacting (G) β-fructofuranosidase with sucrose and maltitol (contact step), wherein the (G) β-fructofuranosidase has a sequence identity of 60% or more with the amino acid sequence set forth in SEQ ID NO: 1 and has β-fructofuranosidase activity.

According to the fructosylation method of the present invention, there is also provided a method for fructosylating maltitol, comprising the step of contacting a (H) polypeptide comprising a part of the (G) β-fructofuranosidase with sucrose and maltitol (contact step), wherein the (H) polypeptide comprises an enzymatically active moiety of the amino acid sequence of β-fructofuranosidase having the amino acid sequence set forth in SEQ ID NO: 1 and has β-fructofuranosidase activity.

According to the fructosylation method of the present invention, there is further provided a method for fructosylating maltitol, comprising the step of contacting (I) a culture of a transformant comprising either or both of the (G) β-fructofuranosidase and the (H) polypeptide, with sucrose and maltitol (contact step), wherein the (I) culture of the transformant is a culture of a transformant obtained by introducing into a host an expression vector comprising a polynucleotide encoding the (G) β-fructofuranosidase or a polynucleotide encoding the (H) polypeptide.

The fructosylation method of the present invention can be implemented according to the descriptions about the production method of the present invention, in addition to the above description.

### <<β-Fructofuranosidase>>

According to the present invention, there is also provided use of β-fructofuranosidase for producing a fructosylated maltitol, wherein the β-fructofuranosidase has a sequence identity of 60% or more with the amino acid sequence set forth in SEQ ID NO: 1 and has β-fructofuranosidase activity. The use of the present invention can be implemented according to the descriptions about the production method of the present invention, in addition to the above description.

The fructosylation method of the present invention can be implemented according to the descriptions about the production method of the present invention, in addition to the above description.

### EXAMPLES

The present invention will be described in more detail by way of the following examples, but is not limited to these examples.

### Example 1: Study (1) on fructosylation of sugar alcohol

In Example 1, fructosylation of each sugar alcohol was studied using β-fructofuranosidase.

### (1) Method

Using β-fructofuranosidase (AfBFFase) having the amino acid sequence (SEQ ID NO: 1) derived from *Aspergillus fijiensis* (*Aspergillus fijiensis* ATCC20611) indicated in Table 2, fructosylation of each of the sugar alcohols indicated in Table 3 was studied under the reaction conditions indicated in Table 4 for the sugar alcohols. For each reaction solution, the components of each sugar were analyzed under the analytical conditions indicated in Table 5 using high performance liquid chromatography (HPLC). For results of the component analysis, the proportion (% (w/w)) of each sugar to all sugars was calculated based on the peak area of HPLC.

**[Table 2]**

| |
|---|
| Table 2: Amino acid sequence (SEQ ID NO: 1) of AfBFFase |
| MKLTTTTLALATGAAAAEASYHLDTTAPPPTNLSTLPNNTLFHVWRPRAHILPA EGQIGDPCAHYTDPSTGLFHVGFLHDGDGIAGATTANLATYTDTSDNGSFLIQP GGKNDPVAVFDGAVIPVGVNNTPTLLYTSVSFLPIHWSIPYTRGSETQSLAVARD GGRRFDKLDQGPVIADHPFAVDVTAFRDPFVFRSAKLDVLLSLDEEVARNETAV QQAVDGWTEKNAPWYVAVSGGVHGVGPAQFLYRQNGGNASEFQYWEYLGE WWQEATNSSWGDEGTWAGRWGFNFETGNVLFLTEEGHDPQTGEVFVTLGTE GSGLPIVPQVSSIHDMLWAAGEVGVGSEQEGAKVEFSPSMAGFLDWGFSAYAA AGKVLPASSAVSKTSGVEVDRYVSFVWLTGDQYEQADGFPTAQQGWTGSLLL PRELKVQTVENVVDNELVREEGVSWVVGESDNQTARLRTLGITIARETKAALL |

| |
|---|
| ANGSVTAEEDRTLQTAAVVPFAQSPSSKFFVLTAQLEFPASARSSPLQSGFEILAS ELERTAIYYQFSNESLVVDRSQTSAAAPTNPGLDSFTESGKLRLFDVIENGQEQ VETLDLTVVVDNAVVEVYANGRFALSTWARSWYDNSTQIRFFHNGEGEVQFR NVSVSEGLYNAWPERN |

| |
|---|
| *The 1^{st} to 19^{th} amino acids (underlined portion in the sequence) indicate a signal sequence. |

**[Table 3]**

| | |
|---|---|
| Table 3: Sugar alcohol | |
| Sorbitol | Monosaccharide sugar alcohols |
| Xylitol | |
| Erythritol | |
| Galactitol | |
| Mannitol | |
| Lactitol | Disaccharide sugar alcohols |
| Maltitol | |
| Palatinit | |

**[Table 4]**

| | |
|---|---|
| Table 4: Reaction conditions | |
| AfBFFase | 1.0 µg |
| Sucrose | 1.0 M |
| Sugar alcohol* | 1.0 M |
| Solvent | 300 µL (50 mM sodium phosphate |

| | |
|---|---|
| | buffer, pH 5.5) |
| Reaction temperature | 50°C |
| Reaction time | 48 hr |

| | |
|---|---|
| *Any one of sugar alcohols indicated in Table 3 | |

**[Table 5]**

| Table 5: Analytical conditions | |
|---|---|
| Apparatus | · DGU-20A3 prominence DEGASSER (SHIMADZU CORPORATION) |
| | · LC-20AD prominence LIQUID CHROMATOGRAPH (SHIMADZU CORPORATION) |
| | · SIL-20A prominence AUTO SAMPLER (SHIMADZU CORPORATION) |
| | · CTO-20A prominence COLUMN OVEN (SHIMADZU CORPORATION) |
| | · RID-10A REFRACTIVE INDEX DETECTOR (SHIMADZU CORPORATION) |
| Column | ULTRON AF-HILIC-CD 250 × 4.6 (Shinwa Chemical Industries Ltd.) |
| Column temperature | 30°C |
| Mobile phase | CH₃CN:H₂O = 74.1:25.9 |
| Flow rate | 0.6 mL/min |

| | |
|---|---|
| Injection volume of reaction solution | 6.0 µL |

### (2) Result

The results were as shown in Tables 6 to 9 and FIGS. 1 to 4. It was confirmed that, when maltitol was used as a disaccharide alcohol, maltitol to which one molecule of fructose was bound (F-Mal) and maltitol to which one molecule of fructose was bound (F-Mal) were produced as fructosylated maltitol (Table 6, FIG. 1). On the other hand, when lactitol and Palatinit were used as the disaccharide alcohol, fructosylated lactitol and fructosylated Palatinit were not produced (Tables 7 and 8, and FIGS. 2 and 3). Also, when xylitol was used as a monosaccharide alcohol, no fructosylated xylitol was produced (Table 9, FIG. 4). Similarly, no fructosylated sugar alcohols were produced for monosaccharide alcohols other than xylitol (data not shown). These results demonstrated that AfBFFase fructosylates maltitol in a substrate-specific manner, producing fructosylated maltitol. Although not bound by the following theory, AfBFFase was reactive with maltitol but was not reactive with Palatinit having the same closed ring sugar, and therefore it is speculated that a reducing sugar moiety of maltitol is involved in the contact of AfBFFase. In addition, since lactitol has galactose as a closed ring sugar moiety unlike sucrose and maltitol, it is presumed that glucose is specifically involved in the contact of AfBFFase with maltitol.

**[Table 6]**

| Table 6: Proportion (% (w/w)) of each sugar to all sugars in reaction solution when maltitol was used | | | | | | | | |
|---|---|---|---|---|---|---|---|---|
| | Gul | Suc | Mal | Trisacc haride FOS | F-Mal | Tetrasa ccharid e FOS | F-F-Mal | Pentasa ccharid e FOS |
| | (Monos acchari de) | (Disacc haride) | (Disacc haride) | | (Trisac charide ) | | (Tetras acchari de) | |
| Suc + Mal | 19.6 | 4.6 | 49.6 | 5.1 | 8.0 | 10.2 | 2.9 | 0.1 |
| Suc | 39.7 | 11.7 | - | 13.9 | - | 20.8 | - | 13.9 |

| | | | | | | | | |
|---|---|---|---|---|---|---|---|---|
| (Abbreviations) Gul: glucose, Suc: sucrose, Mal: maltitol, FOS: fructooligosaccharide, F-Mal: maltitol to which one molecule of fructose was bound (fructosylated maltitol), F-F-Mal: maltitol to which two molecules of fructose were bound (fructosylated maltitol), -: not detectable (peak area of HPLC was too small to calculate) | | | | | | | | |

**[Table 7]**

| Table 7: Proportion (% (w/w)) of each sugar to all sugars in reaction solution when lactitol was used | | | | | | | | |
|---|---|---|---|---|---|---|---|---|
| | Gul | Suc | Lac | Trisacc haride FOS | F-Lac | Tetrasa ccharid e FOS | F-F-Lac | Pentasa ccharid e FOS |
| | (Monos acchari de) | (Disacc haride) | (Disacc haride) | | (Trisac charide ) | | (Tetras acchari de) | |
| Suc + | 18.2 | 5.7 | 55.9 | 9.0 | - | 7.9 | - | 3.3 |

| | | | | | | | | |
|---|---|---|---|---|---|---|---|---|
| Lac | | | | | | | | |
| Suc | 39.7 | 11.7 | - | 13.9 | - | 20.8 | - | 13.9 |

| | | | | | | | | |
|---|---|---|---|---|---|---|---|---|
| (Abbreviations) Gul: glucose, Suc: sucrose, Lac: lactitol, FOS: fructooligosaccharide, F-Lac: lactitol to which one molecule of fructose was bound (fructosylated lactitol), F-F-Lac: lactitol to which two molecules of fructose were bound (fructosylated lactitol), -: not detectable (peak area of HPLC was too small to calculate) | | | | | | | | |

**[Table 8]**

| Table 8: Proportion (% (w/w)) of each sugar to all sugars in reaction solution when Palatinit was used | | | | | | | | |
|---|---|---|---|---|---|---|---|---|
| | Gul | Suc | Pal | Trisacc haride FOS | F-Pal | Tetrasa ccharid e FOS | F-F-Pal | Pentasa ccharid e FOS |
| | (Monos acchari de) | (Disacc haride) | (Disacc haride) | | (Trisac charide ) | | (Tetras acchari de) | |
| Suc + Pal | 17.9 | 4.9 | 67.0 | 5.7 | - | 4.3 | - | 0.7 |
| Suc | 39.7 | 11.7 | - | 13.9 | - | 20.8 | - | 13.9 |

| | | | | | | | | |
|---|---|---|---|---|---|---|---|---|
| (Abbreviations) Gul: glucose, Suc: sucrose, Pal: Palatinit, FOS: fructooligosaccharide, F-Pal: Palatinit to which one molecule of fructose was bound (fructosylated Palatinit), F-F-Pal: Palatinit to which two molecules of fructose were bound (fructosylated Palatinit), -: not detectable (peak area of HPLC was too small to calculate) | | | | | | | | |

**[Table 9]**

| Table 9: Proportion (% (w/w)) of each sugar to all sugars in reaction solution when xylitol was used | | | | | | | |
|---|---|---|---|---|---|---|---|
| | Gul + Xyl | Suc | Trisacch aride FOS | F-Xyl | Tetrasac charide FOS | F-F-Xyl | Pentasac charide FOS |
| | (Monosa ccharide ) | (Disacch aride) | | (Trisacc haride) | | (Tetrasac charide) | |
| Suc + Xyl | 53.6 | 10.4 | 10.4 | - | 13.9 | - | 11.7 |
| Suc | 39.7 | 11.7 | 13.9 | - | 20.8 | - | 13.9 |

| | | | | | | | |
|---|---|---|---|---|---|---|---|
| (Abbreviations) Gul: glucose, Xyl: xylitol, Suc: sucrose, FOS: fructooligosaccharide, F-Xyl: xylitol to which one molecule of fructose was bound (fructosylated xylitol), F-F-Xyl: xylitol to which two molecules of fructose were bound (fructosylated xylitol), -: not detectable (peak area of HPLC was too small to calculate) | | | | | | | |

### Example 2: Study (2) on fructosylation of sugar alcohol

In Example 2, the influence of the amount of maltitol as the substrate on the production amount of fructosylated maltitol in the fructosylation of maltitol with β-fructofuranosidase was studied.

### (1) Method

Using the β-fructofuranosidase (AfBFFase) having the amino acid sequence (SEQ ID NO: 1) derived from *Aspergillus fijiensis* (*Aspergillus fijiensis* ATCC20611) indicated in Table 2, the influence on the production amount of fructosylated maltitol was studied under the reaction conditions at each maltitol concentration indicated in Table 10. For each reaction solution, the components of each sugar were analyzed under the analytical conditions indicated in Table 11 using high performance liquid chromatography (HPLC). For results of the component analysis, the proportion (% (w/w)) of each sugar to all sugars and the proportion (% (w/w)) of each FOS to all FOSs were calculated based on the peak area of HPLC.

**[Table 10]**

| | |
|---|---|
| Table 10: Reaction conditions | |
| AfBFFase | 1.0 µg |
| Sucrose:maltitol* | 1:1 (1.0 M:1.0 M), 1:2 (0.67 M:1.33 M) or 1:3 (0.5 M:1.5 M) |
| Solvent | 300 µL (50 mM sodium phosphate buffer, pH 5.5) |
| Reaction temperature | 50°C |
| Reaction time | 48 hr |

| | |
|---|---|
| *The ratio of sucrose to maltitol was adjusted so that the total concentration of them was 2 M. | |

**[Table 11]**

| | |
|---|---|
| Table 11: Analytical conditions | |
| Apparatus | · DGU-20A3 prominence DEGASSER (SHIMADZU CORPORATION) |
| | · LC-20AD prominence LIQUID CHROMATOGRAPH (SHIMADZU CORPORATION) |

| | |
|---|---|
| | · SIL-20A prominence AUTO SAMPLER (SHIMADZU CORPORATION) |
| | · CTO-20A prominence COLUMN OVEN (SHIMADZU CORPORATION) |
| | · RID-10A REFRACTIVE INDEX DETECTOR (SHIMADZU CORPORATION) |
| Column | ULTRON AF-HILIC-CD 250 × 4.6 (Shinwa Chemical Industries Ltd.) |
| Column temperature | 30°C |
| Mobile phase | CH₃CN:H₂O = 74.1:25.9 |
| Flow rate | 0.6 mL/min |
| Injection volume of reaction solution | 6.0 µL |

### (2) Result

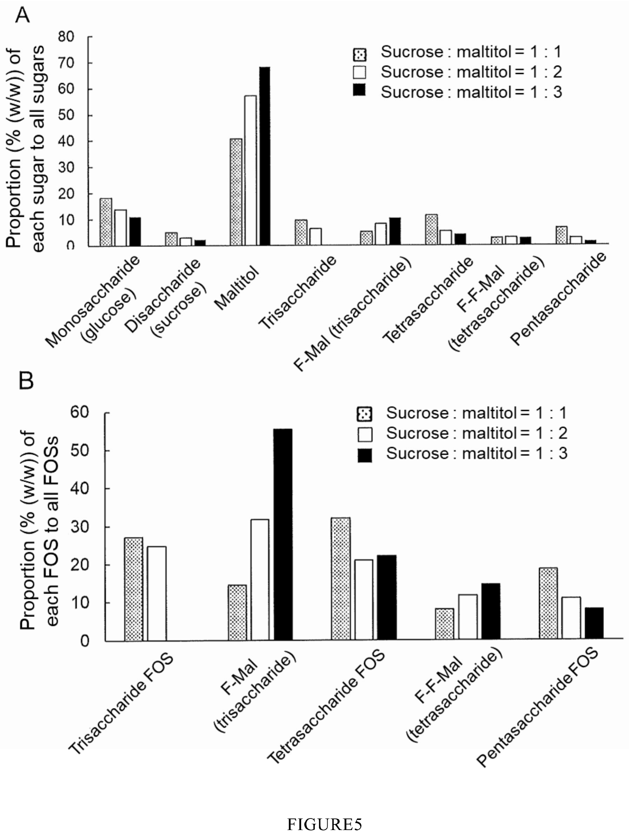

The results were as shown in Tables 12 to 13 and FIG. 5. It was confirmed that, when the concentration of maltitol was increased (ratios of maltitol to sucrose of 1:1, 1:2, and 1:3), the production amount of fructosylated maltitol (F-Mal) to which one molecule of fructose was bound increased in a concentration-dependent manner (Tables 12 and 13 and FIG. 5). These results demonstrated that fructosylation of maltitol with AfBFFase is concentration-dependent. In Table 12, the proportion of maltitol to which two molecules of fructose were bound (F-F-Mal) to all sugars is lower when the ratio of maltitol to sucrose is 1:3 than those when the ratios are 1:1 and 1:2. This is presumably due to depletion of sucrose, which is a source of fructose. On the other hand, in Table 13, the proportion of FF-Mal to all FOSs increases as the ratio of maltitol to sucrose increases from 1:1 to 1:2 and further to 1:3. This is presumably because, when sucrose is depleted, trisaccharide FOS and tetrasaccharide FOS function as donors to supply fructose, whereas F-F-Mal does not function as a donor to supply fructose, resulting in a relative increase in proportion to all FOSs.

**[Table 12]**

| Table 12: Proportion (% (w/w)) of each sugar to all sugars when maltitol at each concentration was used | | | | | | | | |
|---|---|---|---|---|---|---|---|---|
| Suc: Mal | Gul | Suc | Mal | Trisacc haride FOS | F-Mal | Tetrasa ccharid e FOS | F-F-Mal | Pentasa ccharid e FOS |
| | (Monos acchari de) | (Disacc haride) | (Disacc haride) | | (Trisac charide ) | | (Tetras acchari de) | |
| 1:1 | 18.3 | 5.11 | 40.8 | 9.67 | 5.19 | 11.4 | 2.84 | 6.63 |
| 1:2 | 13.8 | 3.11 | 57.2 | 6.43 | 8.23 | 5.43 | 3.04 | 2.82 |
| 1:3 | 11.0 | 2.22 | 68.1 | - | 10.4 | 4.14 | 2.73 | 1.51 |

| | | | | | | | | |
|---|---|---|---|---|---|---|---|---|
| (Abbreviations) Gul: glucose, Suc: sucrose, Mal: maltitol, FOS: fructooligosaccharide, F-Mal: maltitol to which one molecule of fructose was bound (fructosylated maltitol), F-F-Mal: maltitol to which two molecules of fructose were bound (fructosylated maltitol), -: not detectable (peak area of HPLC was too small to calculate) | | | | | | | | |

**[Table 13]**

| Table 13: Proportion (% (w/w)) of each FOS to all FOSs when maltitol at each concentration was used | | | | | |
|---|---|---|---|---|---|
| Suc: Mal | Trisaccharide FOS | F-Mal | Tetrasacchari de FOS | F-F-Mal | Pentasacchari de FOS |
| | | (Trisaccharid e) | | (Tetrasacchar ide) | |
| 1:1 | 27.5 | 14.5 | 31.9 | 7.94 | 18.5 |
| 1:2 | 24.8 | 31.7 | 20.9 | 11.7 | 10.9 |
| 1:3 | - | 55.3 | 22.1 | 14.5 | 8.05 |

| | | | | | |
|---|---|---|---|---|---|
| (Abbreviations) Gul: glucose, Suc: sucrose, Mal: maltitol, FOS: fructooligosaccharide, F-Mal: maltitol to which one molecule of fructose was bound (fructosylated maltitol), F-F-Mal: maltitol to which two molecules of fructose were bound (fructosylated maltitol), -: not detectable (peak area of HPLC was too small to calculate) | | | | | |

### Example 3: Study (3) on fructosylation of sugar alcohol

Example 3 illustrates a production example of fructosylation of maltitol with β-fructofuranosidase.

### (1) Method

Using the β-fructofuranosidase (AfBFFase) having the amino acid sequence (SEQ ID NO: 1) derived from *Aspergillus fijiensis* (*Aspergillus fijiensis* ATCC20611) indicated in Table 2, fructosylated maltitol was produced according to the following procedures (i) to (iii).
(i) The reaction composition liquid indicated in Table 14 was prepared.
(ii) From the composition liquid, 150 µL was collected and subjected to a test under the reaction conditions indicated in Table 15.
(iii) The reaction solution at each reaction time was diluted 10-fold with distilled water and subjected to component analysis by RI-HPLC. The analytical conditions are indicated in Table 16.

**[Table 14]**

| Table 14: Reaction composition liquid | |
|---|---|
| AfBFFase | 20 mg (14.70 million U/g) |
| Sucrose | 133 g |
| Maltitol | 229 g |
| Solvent | 500 mL (50 mM sodium phosphate buffer, pH 5.5) |

**[Table 15]**

| Table 15: Reaction conditions | |
|---|---|
| Reaction temperature | 50°C |
| Reaction time | 0 hr, 2 hr, 4 hr, 6 hr, 8 hr, 10 hr, 12 hr, 24 hr, 26 hr, 28 hr, 30 hr, 32 hr, 34 hr, 48 hr, or 54 hr |

**[Table 16]**

| Table 16: Analytical conditions | |
|---|---|
| Apparatus | •SCL-10A SYSTEM CONTROLLER (SHIMADZU CORPORATION) |
| | •LC-20AT prominence LIQUID CHROMATOGRAPH (SHIMADZU CORPORATION) |

| | |
|---|---|
| | •RID-10A REFRACTIVE INDEX DETECTOR (SHIMADZU CORPORATION) |
| | •SIL-10A AUTO INJECTOR (SHIMADZU CORPORATION) |
| | •CTO-10AS VP COLUMN OVEN (SHIMADZU CORPORATION) |
| Column | HILICpak VG-50 4E4.6mmID×250m ml (Shodex) |
| Column temperature | 45°C |
| Mobile phase | CH₃CN:H₂O = 73:27 |
| Flow rate | 1.0 mL/min |
| Injection volume of reaction solution | 10 µL |

### (2) Result

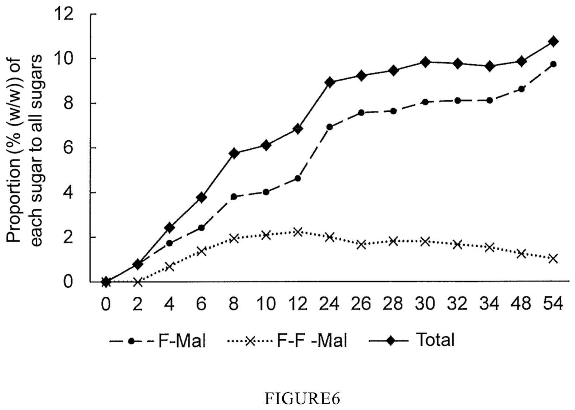

The results were as shown in Table 17 and FIG. 6. The proportion of fructosylated maltitol to all sugars increased with the reaction time for the maltitol to which one molecule of fructose was bound (F-Mal), and the total amount of F-Mal and the maltitol to which two molecules of fructose were bound (FF-Mal) reached a production amount of more than 10% after 54 hours.

**[Table 17]**

| Table 17: Proportion (% (w/w)) to all sugars at each reaction time | | | | | | | | |
|---|---|---|---|---|---|---|---|---|
| Time | 0 | 2 | 4 | 6 | | 8 | 10 | 12 |
| F-Mal | 0 | 0.79 | 1.73 | 2.41 | | 3.80 | 4.01 | 4.62 |

| | | | | | | | | | |
|---|---|---|---|---|---|---|---|---|---|
| F-F-Mal | 0 | 0 | 0.69 | 1.37 | | 1.94 | 2.09 | 2.23 | |
| Total | 0 | 0.79 | 2.42 | 3.78 | | 5.74 | 6.1 | 6.85 | |

**Table 17: Proportion (% (w/w)) to all sugars at each reaction time (continued)**

| Time | 24 | 26 | 28 | 30 | 32 | 34 | 48 | 54 |
|---|---|---|---|---|---|---|---|---|
| F-Mal | 6.92 | 7.56 | 7.63 | 8.03 | 8.10 | 8.11 | 8.61 | 9.72 |
| F-F-Mal | 2.00 | 1.66 | 1.81 | 1.79 | 1.66 | 1.53 | 1.24 | 1.02 |
| Total | 8.92 | 9.22 | 9.44 | 9.82 | 9.76 | 9.64 | 9.85 | 10.7 |

### Example 4: Study (4) on fructosylation of sugar alcohol

Example 4 illustrates a production example of fructosylation of maltitol with β-fructofuranosidase.

Using the β-fructofuranosidase (AfBFFase) having the amino acid sequence (SEQ ID NO: 1) derived from Aspergillus fijiensis (Aspergillus fijiensis ATCC20611) indicated in Table 2, fructosylated maltitol can be produced according to the following procedures (i) to (iii).
(i) The reaction composition liquid indicated in Table 18 is prepared.
(ii) From the composition liquid, 150 µL is collected and subjected to a test under the reaction conditions indicated in Table 19.
(iii) The reaction solution at each reaction time is diluted 10-fold with distilled water and subjected to component analysis by RI-HPLC. The analytical conditions are indicated in Table 20.

**[Table 18]**

| Table 18: Reaction composition liquid | |
|---|---|
| AfBFFase | 80 mg |
| Sucrose | 452 g |
| Maltitol | 916 g |
| Solvent | 2000 mL (50 mM sodium phosphate buffer, pH 5.5) |

**[Table 19]**

| Table 19: Reaction conditions | |
|---|---|
| Reaction temperature | 50°C |
| Reaction time | 0 hr, 2 hr, 4 hr, 8 hr, 12 hr, 24 hr, 30 hr, or 48 hr |

**[Table 20]**

| Table 20: Analytical conditions | |
|---|---|
| Experimental instrument | •SCL-10A SYSTEM CONTROLLER (SHIMADZU CORPORATION) |
| | •LC-20AT prominence LIQUID CHROMATOGRAPH (SHIMADZU CORPORATION) |
| | •RID-10A REFRACTIVE INDEX DETECTOR (SHIMADZU CORPORATION) |
| | •SIL-10A AUTO INJECTOR (SHIMADZU CORPORATION) |
| | •CTO-10AS VP COLUMN OVEN (SHIMADZU CORPORATION) |
| Column | HILICpak VG-50 4E4.6mmID×250m ml (Shodex) |

| | |
|---|---|
| Column temperature | 45°C |
| Mobile phase | CH₃CN:H₂O = 73:27 |
| Flow rate | 1.0 mL/min |
| Injection volume of reaction solution | 10 µL |

### Example 5: Study (5) on fructosylation of sugar alcohol

In Example 5, the structure of maltitol (F-Mal) to which one molecule of fructose was bound was determined by NMR analysis.

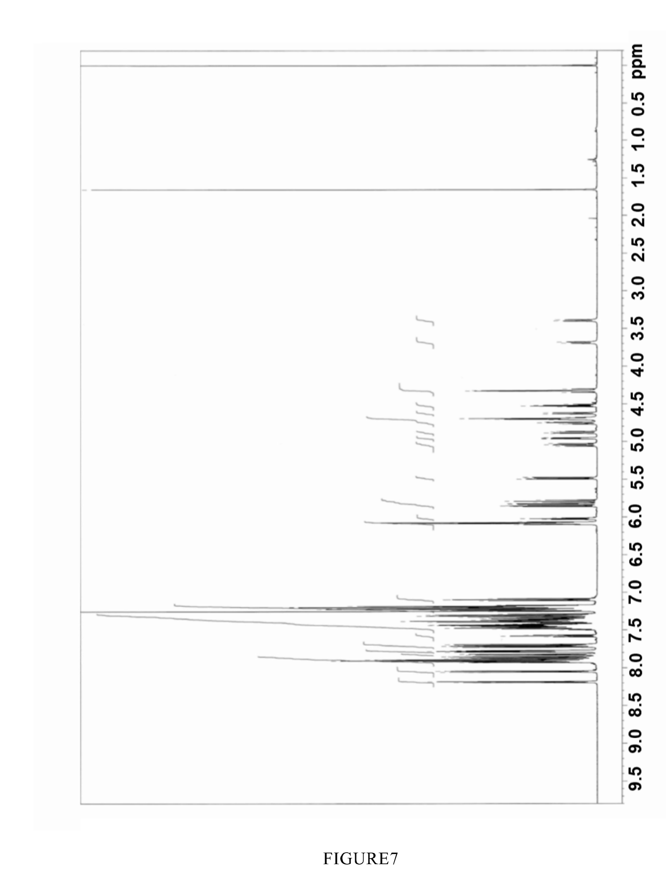

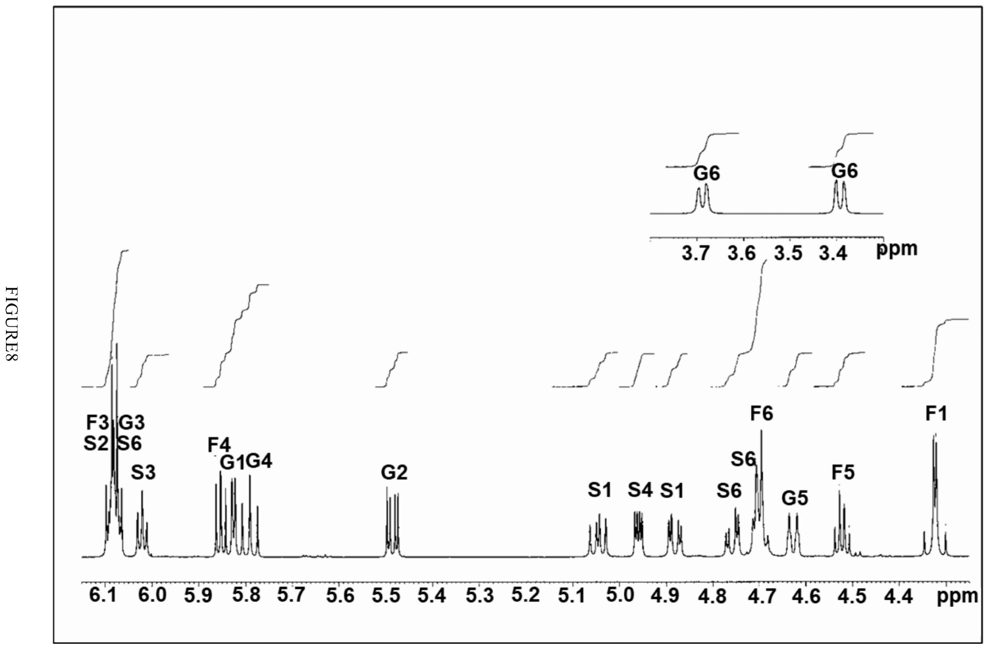

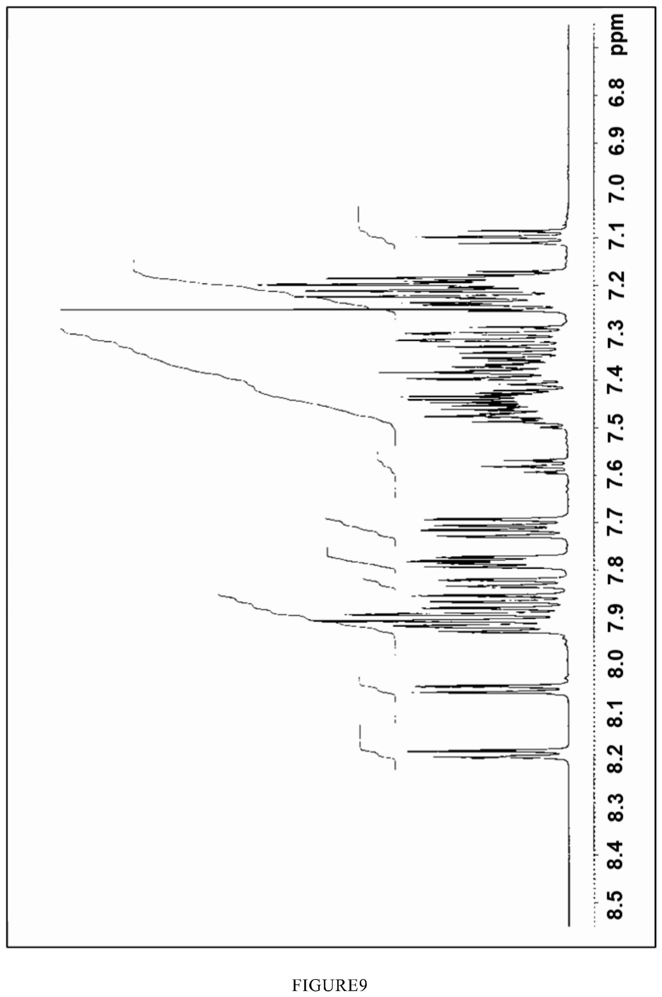

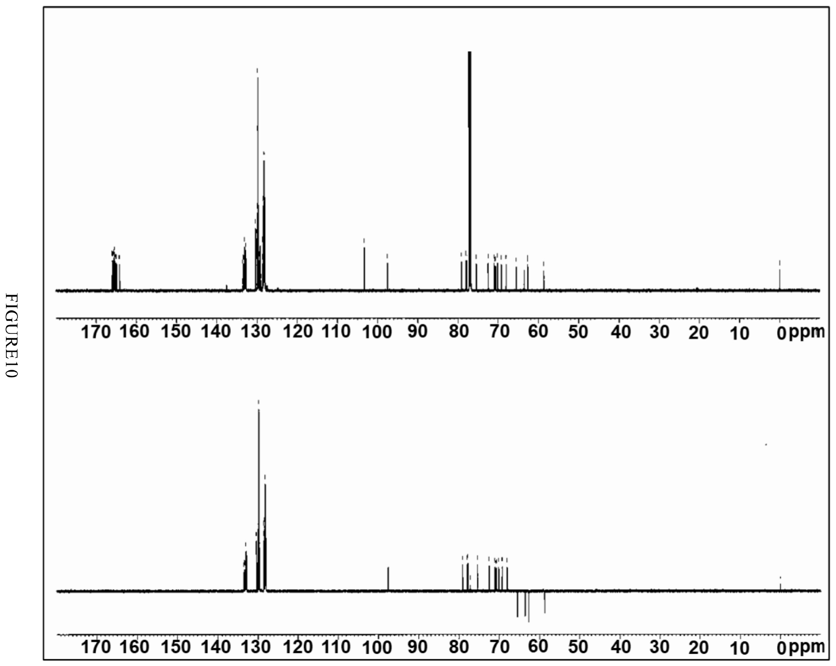

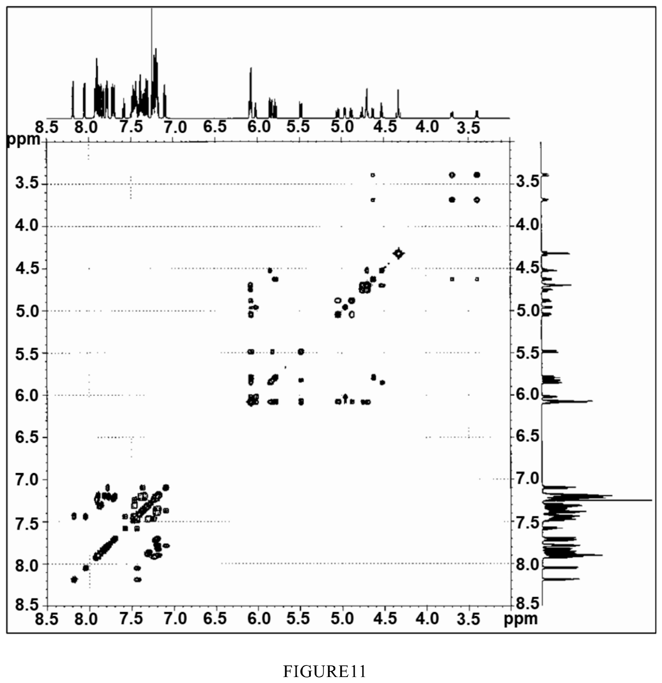

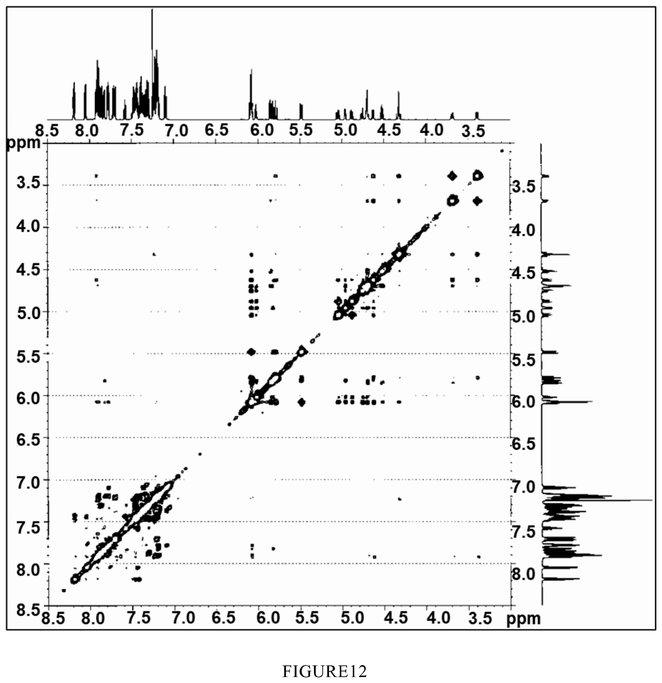

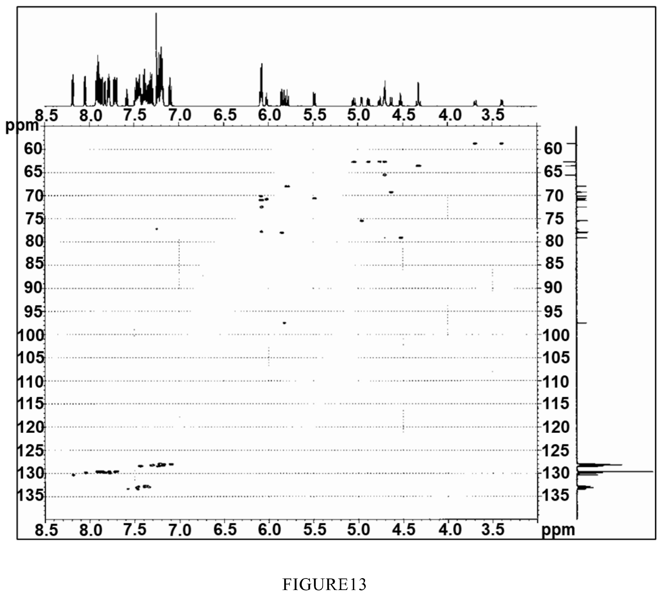

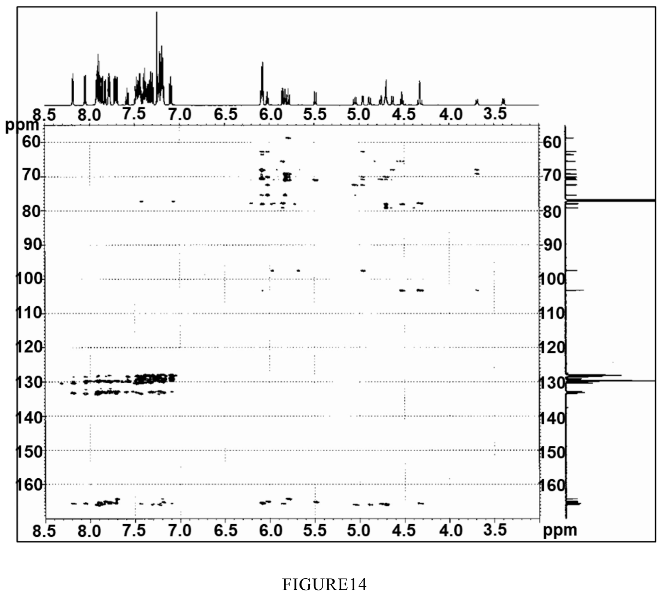

A mixture containing F-Mal (120 mg, 0.237 mmol) was dissolved in dichloromethane (10 ml). The solution was cooled to 0°C, and benzoyl chloride (551 µL, 4.74 mmol), triethylamine (991 µL, 7.11 mmol) and 4-dimethylaminopyridine (145 mg, 1.19 mmol) were added thereto, followed by stirring at room temperature for 24 hours to cause a reaction. Next, 1 M hydrochloric acid was added to stop the reaction, ethyl acetate was added, and the organic layer was washed successively with water and saturated saline, and dried over sodium sulfate. The desiccant was filtered off, and the solvent was distilled off under reduced pressure. The residue was subjected to column chromatography (silica gel, hexane/ethyl acetate = 2/1 to 1/1) and subsequently to gel filtration chromatography (chloroform, 3.5 ml/min) to obtain a yellow amorphous substance containing F-Mal^{(-OBz)}. The yellow amorphous substance was purified by high performance liquid chromatography (silica gel, hexane/ethyl acetate = 4/3, 3.5 ml/min) to obtain F-Mal^{(-OBz)} (154 mg, 37% yield) as a white amorphous substance. NMR analysis of F-Mal^{(-OBz)} (Bruker Avance III 600, Bruker) was performed to obtain a ¹H NMR spectrum, a ¹³C NMR spectrum, a COSY NMR spectrum, a NOESY NMR spectrum, an HSQC NMR spectrum and an HMBC NMR spectrum. Peaks of each NMR spectrum are shown in FIGS. 7 to 14.

¹H NMR (600 MHz, CDCl₃): δ 8.20-8.18 (m, 2H), 8.06-8.04 (m, 2H), 7.93-7.85 (m, 10H), 7.84-7.82 (m, 2H), 7.80-7.77 (m, 4H), 7.73-7.69 (m, 4H), 7.60-7.57 (m, 1H), 7.50-7.29 (m, 20H), 7.25-7.17 (m, 15H), 7.11-7.08 (m, 2H), 6.09-6.07 (m, 4H, G3+S2+S5+F3), 6.02 (dd, J = 6.0, 5.4 Hz, 1H, S3), 5.85 (dd, J = 6.6, 6.0 Hz, 1H, F4), 5.83 (d, J = 3.6 Hz, 1H, G1), 5.79 (dd, J = 10.2, 9.6 Hz, 1H, G4), 5.49 (dd, J = 10.2, 3.6 Hz, 1H, G2), 5.05 (dd, J = 12.0, 8.4 Hz, 1H, S1), 4.96 (dd, J = 6.0, 3.0 Hz, 1H, S4), 4.88 (dd, J = 12.0, 3.6 Hz, 1H, S1), 4.76 (dd, J = 12.0, 3.0 Hz, 1H, S6), 4.72-4.68 (m, 3H, S6+F6), 4.63 (m, 1H, G5), 4.54-4.51 (m, 1H, F5), 4.33 (d, J = 12.0 Hz, 1H, F1), 4.31 (d, J = 12.0 Hz, 1H, F1), 3.69 (dd, J = 9.0, 1.5 Hz, 1H, G6), 3.39 (dd, J = 9.0, 1.5 Hz, 1H, G6). ¹³C NMR (150 MHz, CDCl₃): δ 166.0, 165.9, 165.8, 165.7, 165.54, 165.53, 165.49, 165.4, 165.3, 165.1, 165.0, 164.2, 133.5, 133.4, 133.2, 133.1, 133.03, 133.00, 132.91, 132.86, 132.84, 132.82, 132.80, 130.4, 130.1, 129.93, 129.90, 129.72, 129.70, 129.69, 129.67, 129.45, 129.43, 129.34, 129.27, 129.26, 129.23, 129.17, 129.14, 129.13, 128.6, 128.54, 128.47, 128.45, 128.38, 128.34, 128.30, 128.24, 128.19, 128.11, 128.09, 128.08, 128.0, 103.3, 97.5, 79.1, 78.1, 77.8, 75.5, 72.5, 71.0, 70.8, 70.6, 70.1, 69.2, 68.0, 65.5, 63.6, 62.74, 62.71, 58.7. HRMS (MALDI-TOF): m/z calcd for C₁₀₂H₈₂NaO₂₈ [M+Na]⁺: 1777.49, found: 1777.92.

From the results of analysis of each NMR spectrum, the structure of F-Mal represented by Formula (IV) was determined. Specifically, F-Mal had a structure in which the 6-position of the glucose moiety of maltitol (G6) was bound to the 2-position of fructose (F2). It is considered that there is no contradiction, also from the fact that G6 is shifted to a higher magnetic field and the ppms of the two geminal Hs in G6 are very far apart from each other (because G6 is sandwiched between two rings of glucose and fructose, which causes a very significant steric effects).

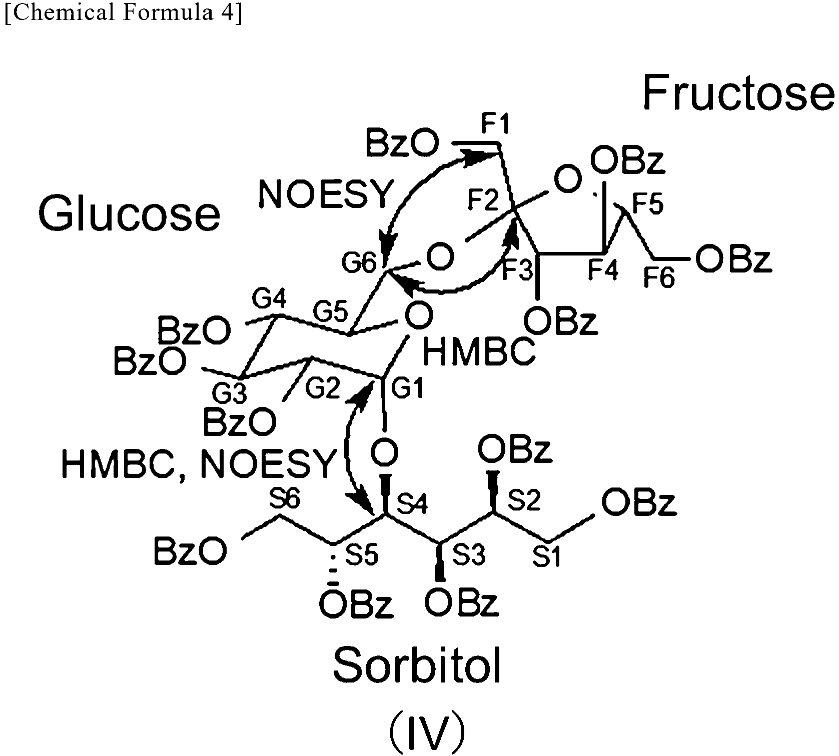

### Industrial Applicability

According to the present invention, it is possible to simply and efficiently produce a fructosylated maltitol. Also, according to the present invention, it is possible to provide a fructosylated maltitol having sweetness and a food containing the same.

## Claims

1. A method for producing a fructosylated maltitol, comprising the step of contacting (A) β-fructofuranosidase or (B) a polypeptide comprising a part thereof, or (C) a culture of a transformant comprising either or both of the β-fructofuranosidase and the polypeptide, with sucrose and maltitol (contact step),
wherein the (A) β-fructofuranosidase has a sequence identity of 60% or more with the amino acid sequence set forth in SEQ ID NO: 1 and has β-fructofuranosidase activity,
wherein the (B) polypeptide comprises an enzymatically active moiety of the amino acid sequence of β-fructofuranosidase having the amino acid sequence set forth in SEQ ID NO: 1 and has β-fructofuranosidase activity, and
wherein the (C) culture of the transformant is a culture of a transformant obtained by introducing into a host an expression vector comprising a polynucleotide encoding the (A) β-fructofuranosidase or a polynucleotide encoding the (B) polypeptide.

2. The method according to claim 1, wherein the β-fructofuranosidase has substrate specificity for maltitol among sugar alcohols.

3. The method according to claim 1 or 2, wherein a ratio of the amount or concentration of the maltitol to the sucrose at the start of contact is 1 or more.

4. The method according to claim 1 or 2, wherein a temperature of the contact step is 40°C to 60°C.

5. The method according to claim 1 or 2, wherein a period of time of the contact step is 8 hours or more.

6. The method according to claim 1 or 2, wherein the host is selected from the group consisting of bacteria, yeasts, molds and filamentous fungi.

7. The method according to claim 1 or 2, wherein the β-fructofuranosidase is derived from a microorganism of the genus *Aspergillus.*

8. The method according to claim 1 or 2, wherein the β-fructofuranosidase is derived from *Aspergillus fijiensis.*

9. An enzyme preparation for use in production of a fructosylated maltitol, comprising β-fructofuranosidase or a polypeptide comprising a part thereof, or a culture of a transformant comprising either or both of the β-fructofuranosidase and the polypeptide,
wherein the β-fructofuranosidase has a sequence identity of 60% or more with the amino acid sequence set forth in SEQ ID NO: 1 and has β-fructofuranosidase activity,
wherein the polypeptide comprises an enzymatically active moiety of the amino acid sequence of β-fructofuranosidase having the amino acid sequence set forth in SEQ ID NO: 1 and has β-fructofuranosidase activity, and wherein the culture of the transformant is a culture of a transformant obtained by introducing into a host an expression vector comprising a polynucleotide encoding the β-fructofuranosidase or a polynucleotide encoding the polypeptide.

10. A fructosylated maltitol which is maltitol to which one or two molecules of fructose is/are bound.

11. The fructosylated maltitol according to claim 10, wherein the maltitol to which one molecule of fructose is bound is represented by Formula (I):

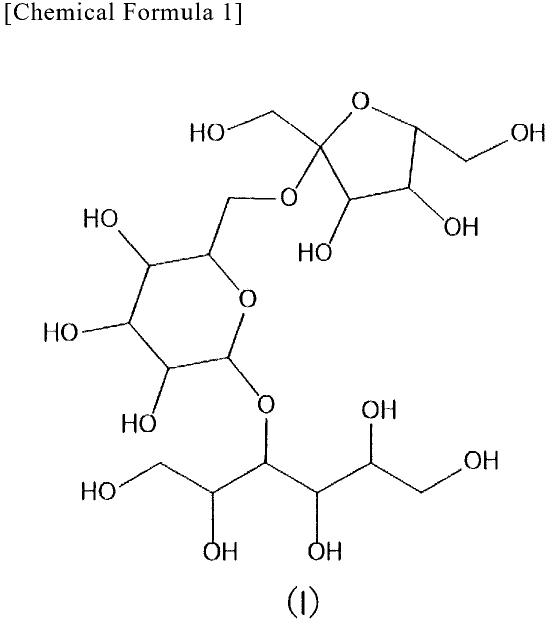

12. The fructosylated maltitol according to claim 10, wherein the maltitol to which two molecules of fructose are bound is represented by Formula (II):

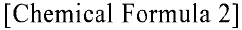

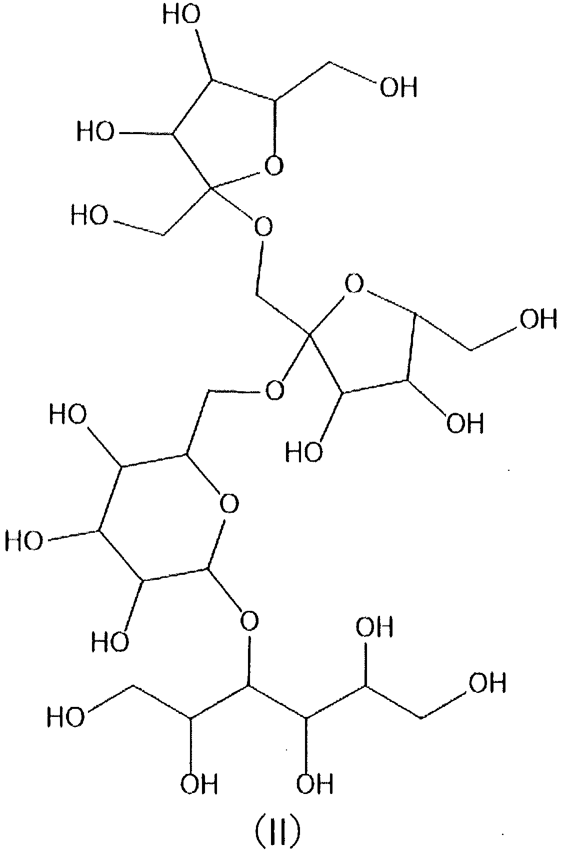

13. An enzyme reaction composition comprising: as an enzyme, (D) β-fructofuranosidase or (E) a polypeptide comprising a part thereof, or (F) a culture of a transformant comprising either or both of the β-fructofuranosidase and the polypeptide; and, as a substrate, sucrose and maltitol,
wherein a proportion of the fructosylated maltitol to all sugars in the composition is 5% or more,
wherein the (D) β-fructofuranosidase has a sequence identity of 60% or more with the amino acid sequence set forth in SEQ ID NO: 1 and has β-fructofuranosidase activity,
wherein the (E) polypeptide comprises an enzymatically active moiety of the amino acid sequence of β-fructofuranosidase having the amino acid sequence set forth in SEQ ID NO: 1 and has β-fructofuranosidase activity, and
wherein the (F) culture of the transformant is a culture of a transformant obtained by introducing into a host an expression vector comprising a polynucleotide encoding the (D) β-fructofuranosidase or a polynucleotide encoding the (E) polypeptide.

14. Use of β-fructofuranosidase for producing a fructosylated maltitol, wherein the β-fructofuranosidase has a sequence identity of 60% or more with the amino acid sequence set forth in SEQ ID NO: 1 and has β-fructofuranosidase activity.
